(19) Europäisches Patentamt
European Patent Office
Office européen des brevets

(11) **EP 3 143 933 B1**

(12) **EUROPEAN PATENT SPECIFICATION**

(45) Date of publication and mention
of the grant of the patent:
**22.08.2018 Bulletin 2018/34**

(51) Int Cl.:
*A61B 5/16* (2006.01)    *A61B 5/0476* (2006.01)
*A61B 5/048* (2006.01)

(21) Application number: **16188795.5**

(22) Date of filing: **14.09.2016**

(54) **METHOD FOR ESTIMATING A MENTAL STATE, IN PARTICULAR A WORKLOAD, AND RELATED APPARATUS**

VERFAHREN ZUR SCHÄTZUNG EINES GEMUTSZUSTANDS UNTER ARBEITSDRUCK UND EINE VERWANDTE VORRICHTUNG

PROCÉDÉ D'ESTIMATION D'UN ÉTAT MENTAL, NOTAMMENT D'UNE CHARGE DE TRAVAIL, ET APPAREIL ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2015 IT UB20153636**

(43) Date of publication of application:
**22.03.2017 Bulletin 2017/12**

(73) Proprietor: **BrainSigns s.r.l.**
**00152 Rome (IT)**

(72) Inventors:
• **Aricò, Pietro**
**00152 Rome (IT)**
• **Borghini, Gianluca**
**00152 Rome (IT)**
• **Di Flumeri, Gianluca**
**00152 Rome (IT)**
• **Babiloni, Fabio**
**00152 Rome (IT)**

(74) Representative: **Scilletta, Andrea**
**IP Sextant s.r.l.**
**Via Antonio Salandra, 18**
**00187 Roma (IT)**

(56) References cited:
**WO-A1-02/00110**        **WO-A1-2009/134205**
**US-A1- 2007 173 733**    **US-A1- 2014 316 230**

**Description**

The present invention concerns a method for estimating a mental state, in particular a workload, of a subject, that allows to make estimations which are precise, efficient, stable and highly reliable over time, without requiring frequent recalibrations.

The present invention further concerns the apparatus configured to execute such method.

It is known that monitoring of the mental state of a subject, such as for instance an emotional state, a cognitive workload, or an attention or concentration level, is of particular interest, especially in safety-critical operative environments where human behaviour is often the least controllable factor, where it is useful at least to estimate the operators' cognitive workload while facing specific operative and emergency conditions. In particular, the mental workload is a measure of the cognitive resources required to process information during a specific task: as cognitive workload increases, maintaining the level of task performance within an acceptable range becomes harder. High cognitive workload may demand more cognitive resources than those available in the human brain, resulting into performance degradation and increase in errors committed.

US2007/0173733 discloses a method of detecting a mental state which includes receiving, in a processor, bio-signals of a subject from one or more bio-signal detectors, and determining in the processor whether the bio-signals represent the presence of a particular mental state in the subject.

As illustrated by E.A. Byrne et al. in "Psychophysiology and adaptive automation", 1996, Biol. Psychol. 42, 249-268, objective measures of mental workload based on biomarkers for evaluating different system design have been proposed in the prior art, in order to appropriately allocate the demand of cognitive resources, to manage the workload level, to minimize errors due to overloads and to intervene on the same systems before the operators could undergo overload conditions. For example, many prior art studies have investigated neurophysiological indexes with reference to the mental states of operators in safety-critical applications, such as vehicle driving (see S. Welke et al. in "Single-Trial Detection of Cognitive Processes for Increasing Traffic Safety", Proceedings of the 21st (Esv) International Technical Conference On The Enhanced Safety Of Vehicles, June 2009, Stuttgart, Germany), industrial environment or security surveillance applications (see B. Venthur et al. in "Novel applications of BCI technology: Psychophysiological optimization of working conditions in industry", 2010 IEEE International Conference on Systems Man and Cybernetics (SMC), IEEE, pp. 417-421), aviation and air traffic controllers' contexts (see B. Willems in "Air traffic control specialist visual scanning II: Task load, visual noise, and intrusions into controlled airspace", 1999, accessible from the web page at the addressht-tp://www.academia.edu/3326249/Air_traffic_control_specialist_visual_
scanning_II_Task_load_visual_noise_and_intrusions_into_controlled_airspace; and G.F. Wilson et al. in "The use of cardiac and eye blink measures to determine flight segment in F4 crews", 1991, Aviat. Space Environ. Med. 62, 959-962).

In such context, several approaches have been proposed in the prior art to evaluate the mental workload, and in particular those based on:

- a subjective evaluation, as disclosed by S.G. Hart in "Development of NASA-TLX (Task Load Index): Results of Empirical and Theoretical Research", 1988, Human Mental Workload, North-Holland, pp. 139-183, or
- a performance evaluation, as disclosed by J.R. Comstock in "MATB - Multi-Attribute Task Battery for human operator workload and strategic behaviour research", 1994, or
- a psychophysiological variables assessment, as disclosed by G. Borghini et al. in "Measuring neurophysiological signals in aircraft pilots and car drivers for the assessment of mental workload, fatigue and drowsiness", 2012 Neurosci. Biobehav. Rev.

Subjective evaluation is a measure assessed by subjective introspections, providing a rate of the perceived workload after the performed task, as the technique called NASA-TLX. Performance evaluation provides a direct relationship between the performance achieved by the subject and the required mental workload (e.g. reaction times, number of lost targets), as disclosed by H.A. Colle et al. in "Double trade-off curves with different cognitive processing combinations: testing the cancellation axiom of mental workload measurement theory", 1999 Hum. Factors 41, 35-50. Finally, the psychophysiological measure consists in the evaluation of the variability (and correlation) of one or more neurophysiological signals (e.g., brain activity, heart activity, ocular activity) with respect to the mental workload required from the subject while performing the task.

In this regard, several prior art studies associate the correlation of spectral power of the electroencephalogram (EEG) with the complexity of the task. For instance, as disclosed by Mogford et al. in "Application of Research Techniques for Documenting Cognitive Processes in Air Traffic Control: Sector Complexity and Decision Making", 1994 (DOT/FAA/CT-TN94/3), Atlantic City International Airport: Federal Aviation Administration Technical Center, an increase of the spectral power in the theta band (i.e., in the frequency range 4-7 Hz), especially over the frontal brain sites, and a decrease of spectral power in the alpha band (i.e., in the frequency range 8-12 Hz), over the parietal brain sites, have been observed when the mental workload increases. An increase in the theta band over occipital sites has also been observed in several prior art studies involving pilots, as those disclosed by C. Dussault et al. in "EEG and ECG changes during selected flight sequences", 2004 Aviat. Space Environ. Med. 75, 889-897, and da T.C. Hankins et al. in "A comparison of heart rate, eye activity, EEG and subjective measures of pilot mental workload during flight", 1998 Aviat. Space Environ. Med. 69, 360-367, where it has been demonstrated that this behaviour is related to an increase of the effort of visual attention towards the flight instruments, whereby such increase in the theta band over occipital sites can be defined as "visual workload".

Although these changes in the EEG spectral power are reproducible across different subjects, their estimations are often slow (more than five minutes in order to highlight differences between different mental workload levels). In order to address this problem, different solutions have been proposed in the prior art. In particular, machine-learning techniques, employing linear and non-linear classifiers, allow to assess the mental workload of subjects in short times (of the order of few seconds), reaching high binary discrimination accuracy (higher than 90%, as disclosed by P. Aricò et al. in "Towards a multimodal bioelectrical framework for the online mental workload evaluation", 36th Annual International Conference of the IEEE Engineering in Medicine and Biology Society (EMBC), 2014, pp. 3001-3004, and by J. Kohlmorgen et al. in "Improving human performance in a real operating environment through real-time mental workload detection", 2007, accessible from the web page at the address http://eprints.pascal-network.org/archive/00003321/. In these approaches, different classifiers, such as for instance the linear discriminant analysis (LDA, disclosed by R.A. Fisher in "The Use of Multiple Measurements in Taxonomic Problems", 1936 Annals of Eugenics 7, 179-188), support vector machines (SVM), and artificial neural network (ANN), have been used to classify the workload into several levels (e.g. low, moderate and high) by using various EEG parameters, with both simple and complex tasks, as disclosed by A. Gevins et al. in "Monitoring working memory load during computer-based tasks with EEG pattern recognition methods", 1998, Hum. Factors, 40, 79-91, by A.R. Nikolaev et al. in "Reproducible EEG alpha-rhythm patterns in solving psychological tasks", 1998 Fiziol. Cheloveka 24, 5-12, and by G.F. Wilson et al., 1991, cited above.

Among the linear classifiers, the stepwise linear discriminant analysis (SWLDA) is an extension of the LDA, which performs a reduction of the space of the features by selecting the most significant ones. Particularly, the SWLDA regression consists in the combination of forward and backward stepwise analyses, where the input features are weighted by using an ordinary least-squares regression to predict the class (i.e. the class label).

The SWLDA method starts by creating an initial model of the discriminant function wherein the most statistically significant feature is added to the model for predicting the target labels:

$$pval_{ij} < \alpha_{ENTER}$$

where $pval_{ij}$ represents the p value (p-value) of the i-th feature at the j-th iteration (in this case the first iteration - in general the iterations are the forward and backward repetitions in the search for features to be included in the model), and $\alpha_{ENTER}$ is an entry significance threshold that is pre-set and possibly adjustable by an operator.

Afterwards, at every new iteration, the i-th feature is added to the model if

$$pval_{ij} < \alpha_{ENTER}$$

If there are no more features satisfying this condition, a backward elimination analysis is performed to remove the least statistically significant feature from the model if

$$pval_{ij} > \alpha_{REMOVE}$$

where $\alpha_{REMOVE}$ is a removal significance threshold that is pre-set and possibly adjustable by an operator.

As disclosed by N.R. Draper et al. in "Applied Regression Analysis", 1998, Third. and., Wiley-Interscience, this process goes on unless there are no more features satisfying the entry condition (with respect to the entry significance threshold $\alpha_{ENTER}$) nor the removal condition (with respect to the removal significance threshold $\alpha_{REMOVE}$), or until a maximum number Iteration$_{MAX}$ of iterations, that is pre-set and possibly adjustable by an operator, is reached.

In principle, it is possible to optimise a SWLDA regression by finely tuning all or some of the three parameters available in the algorithm, namely $\alpha_{ENTER}$, $\alpha_{REMOVE}$ and Iteration$_{MAX}$, for instance through manual calibration by an operator on the basis of the expected characteristics of the data. However, the standard SWLDA algorithm uses

$$\alpha_{ENTER} = 0,05$$

and

$$\alpha_{REMOVE} = 0,1$$

while no constraint is imposed on the Iteration$_{MAX}$ parameter.

In this regard, the values of $\alpha_{ENTER}$ and $\alpha_{REMOVE}$ are set on an empirical basis in the classical SWLDA, mainly depending on the experience related to the type of datum, and 0,05 and 0,1 (assigned to $\alpha_{ENTER}$ and $\alpha_{REMOVE}$, respectively) are standard values, i.e. values accepted in the whole scientific literature. More precisely, the value of 0,05 assigned to $\alpha_{ENTER}$ refers to the know level of statistical significance of 5%, and by setting this value as step, a value of $\alpha_{REMOVE}$ equal to 0,05 incremented of one step (i.e. equal to 0,1) is set in order not to render the SWLDA algorithm too stringent. However, it is also possible to use other values for $\alpha_{ENTER}$ and $\alpha_{REMOVE}$.

In the prior art, there are no clear evidences in relation to which, between linear and non linear classifiers, have better performance in mental workload estimation; by way of example, J.C. Christensen et al., in "The effects of day-to-day variability of physiological data on operator functional state classification", 2012 Neuroimage 59, 57-63, have stated that SVMs with non linear kernel have better performance than a simple LDA, while P.L. Craven et al. in "Cognitive Workload Gauge Development: Comparison of Real-time Classification Methods, Augmented Cognition: Past, Present and Future", 2006, in D. Schmorrow, K. Stanney, and L. Reeves, (Eds) Foundation in Augmented Cognition (2nd and.), pp. 66-74, Arlington, VA, Strategic Analysis, Inc., have stated that linear classifiers have better performance than non linear ones.

As disclosed by F. Aloise et al. in "A comparison of classification techniques for a gaze-independent P300-based brain-computer interface", 2012, Journal of neural engineering 9, 045012, and by D.J. Krusienski in "A comparison of classification techniques for the P300 Speller", 2006, J. Neural Eng. 3, 299-305, the SWLDA method is one of the linear classifiers having best performance. In fact, with respect to other linear methods, it has the advantage of having an automatic extraction of features, so that insignificant terms are statistically removed from the model.

Machine-learning approaches for the estimation of mental states by using physiological data have had a rapid expansion in the last decades. In fact, on the basis of an appropriate training procedure for the extraction of physiological features of interest, these techniques allow to quantify the current mental state (e.g. the mental workload) of a subject with a high time resolution (e.g. few seconds).

A machine learning technique can endow a system with the ability of extracting significant information from considerable amounts of data. Such techniques are widely used in different neuroscience areas. In particular, in the recognition of mental states (such as the mental workload) of a subject, such techniques should be capable to extract from the big amount of physiological data the most significant features closely correlated to the examined mental state. Such features could then be used to assess the mental state of the examined subject in different working-days, whereby the measure should be reliable and stable over time.

However, one of the outstanding problems of the prior art machine learning techniques regards their inability to maintain a high reliability over time (e.g. even across days) without any need for making recalibrations. Also, these techniques may select physiological features not strictly related to the analysed cognitive phenomenon as the most important ones, resulting in a classification not correlated to the mental state of the subject, and thus meaningless.

A classification that has not good performance across different days could be indicative of two issues:

i) the features selected by the classifier on a day change significantly across the other days; in this regard, it has to be understood whether such features are really related to the considered mental state, whether they change because of learning processes (e.g. features related to the mental state of the subject could change over time making the measure inaccurate across several days), or whether the classifier is taking account of features not related to the examined mental state;

ii) the classifier is not well optimised, i.e. it becomes too specific on the unique or spurious differences between classes (i.e. among different difficulty levels of the task) belonging to the training dataset; this phenomenon is known as overfitting: the reduction of features selected by the classifier can mitigate the overfitting and improve the reliability and stability of the classifier over time.

In order to optimise the selection of brain features for training the classifier and for the evaluating mental states, it must be noted that:

- only the physiological features strictly related to the observed phenomenon should be used for training the classifier (e.g. frontal theta and parietal alpha rhythms for the mental workload assessment), and
- among these features, the classifier should select the most significant set that allows to reach the optimum classification over time.

Also, it is important, but it is not at all trivial, to find the right number and type of features to train the classifier, and this depends on the considered problem.

Nowadays, the effects of day-to-day fluctuations in the physiology of the subject have not been thoroughly assessed while subjects are engaged in complex tasks. Different prior art studies have demonstrated that the performance of classifiers in evaluating the different mental workload levels of the subject drastically decrease over subsequent days, as disclosed by A. Burgess et al. in "Individual reliability of amplitude distribution in topographical mapping of EEG", 1993 Electroencephalogr. Clin. Neurophysiol. 86, 219-223, by Christensen et al. in, 2012, cited above, by L.K. McEvoy et al. in "Test-retest reliability of cognitive EEG", 2000 Clin. Neurophysiol. 111, 457-463, by V.E. Pollock et al. in "Reliability of topographic quantitative EEG amplitude in healthy late-middle-aged and elderly subjects", 1991 Electroencephalogr. Clin. Neurophysiol. 79, 20-26, and by M.C. Salinsky et al. in "Test-retest reliability in EEG frequency analysis", 1991 Electroencephalogr. Clin. Neurophysiol. 79, 382-392. Recently, attempts have been made to test the stability of different classifiers for assessing the mental workload of the subject across different days. In these works, all the frequency bands (in the frequency range 0,1 - 100 Hz) have been considered in the training step of the classifier, and for this reason the classifiers were not able to exclusively select the features strictly related to the mental workload. It is evident that the need for daily recalibrations renders the prior art techniques unusable in operative environments.

Also, the SWLDA method above suffers of similar drawbacks. In fact, it is immediate to realize how complex is to properly set up the correct parameters, i.e. $\alpha_{ENTER}$, $\alpha_{REMOVE}$ and Iteration$_{MAX}$, in order to optimise the algorithm. In particular, as disclosed by V.N. Vapnik in "The Nature of Statistical Learning Theory", 2000, Springer New York, New York, NY, the more general the classification training, the higher the reliability of the algorithm over time will be. For example, if the chosen parameters are too selective (i.e. when values of $\alpha_{ENTER}$ and/or $\alpha_{REMOVE}$, and/or Iteration$_{MAX}$ are too much low), the features added to the model will be not sufficient for predicting the target labels with as much accuracy as possible; this phenomenon is known as underfitting, as disclosed by U. von Luxburg et al. in "Statistical Learning Theory: Models, Concepts, and Results", 2008, arXiv:0810.4752 [math, stat]. On the contrary, if the chosen parameters are poorly selective (i.e. when values of $\alpha_{ENTER}$ and/or $\alpha_{REMOVE}$ and/or Iteration$_{MAX}$ are too much high), most features added in the final model could be correlated to spurious differences between classes of the training dataset, which are obviously not generalizable, so that the reliability of the algorithm decreases over time, resulting in overfitting, as disclosed by Vapnik, 2000, cited above.

Therefore, it is an object of the present invention to estimate a mental state of a subject, in particular a workload of a subject, through a classifying technique that allows to make estimations which are precise, efficient, stable and highly reliable over time, without requiring frequent recalibrations, consequently resulting usable in real working environments.

It is specific subject matter of the present invention a method for estimating a mental state, in particular a workload, of a subject, the related apparatus, the related computer program and the computer-readable memory medium storing the same, as defined in the attached independent claims.

Further embodiments of the method and apparatus according to the invention are defined in the attached dependent claims.

The invention is based on an innovative approach of classification, in the following also indicated as automatic-stop Stepwise Linear Discriminant Analysis, or asSWLDA, that is able to automatically optimise the process of selection of the features by using the brain activity of the subject the mental state of whom is to be estimated (e.g. the mental workload), in order to make the performance of classification of the considered mental state stable over time (in particular, over different days, optionally over at least one week). To this end, the invention takes the EEG theta and alpha rhythms into account, because of their strong correlation with the mental workload (thus neglecting the features which are outside the EEG theta and alpha rhythms and which are not correlated to the mental workload, e.g. due to artefacts due to movements, and hence which could be specific of the single recording, consequently impairing stability of the method over time), and it introduces an automatic stop in the standard SWLDA method that automatically interrupts the process of selection of the features as soon as an optimal number of features have been added to the model.

In particular, the invention is capable to identify subjective EEG features directly correlated to the mental workload, which remain sufficiently stable across several days, while a subject is performing a task at two or more difficulty levels.

The numerous advantages offered by the asSWLDA technique and, consequently, by the method according to the invention are evident.

First of all, it maintains high performance over several days without any need for recalibration.

Moreover, determination of the optimal number of features not only allows to reduce the features selected by the classifier, drastically mitigating the overfitting phenomenon, but it further allows to add to the model a number of features sufficient to drastically mitigate also the underfitting phenomenon.

Furthermore, the asSWLDA technique and the method according to the invention are capable to significantly differentiate different levels of mental workload from each other, related to corresponding different levels of difficulty of a task performed by a subject the mental workload of whom is to be estimated.

The inventors have verified the aforementioned advantages through some tests, the results of which have shown a high increase of reliability of classification of the mental workload with respect to the standard SWLDA technique.

In particular, the method according to the invention may be used in real working environments, wherein for instance pilots, or other types of subjects, such as air traffic controller, drivers or operators in other contexts where conditions of high stress could cause a critical drop of performance with dangerous consequences.

The present invention will be now described, by way of illustration and not by way of limitation, according to its preferred embodiments, by particularly referring to the Figures of the annexed drawings, in which:

Figure 1 shows a schematic block diagram of a preferred embodiment of the method according to the invention; and
Figure 2 shows three graphs obtainable during execution of the method of Figure 1.

In the following of the present description, reference will be mainly made to a specific application of a preferred embodiment of the method (and related apparatus) according to the invention dedicated to the estimation of the cognitive workload of subjects while facing facing specific operative and emergency conditions.

However, it must be noted that the method and related apparatus according to the invention may be applied for estimating any mental state, even different from the workload, such as an emotional state or an attention or concentration level, still remaining within the scope of protection of the present invention as defined in the attached claims.

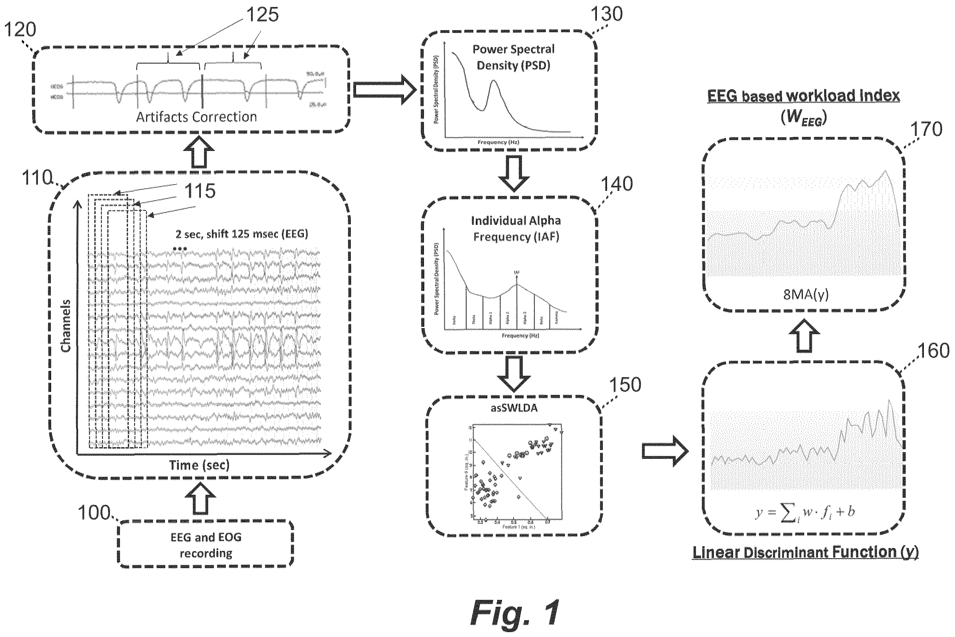

Figure 1 shows the steps of the preferred embodiment of the method for estimating a mental workload according to the invention by means of the surface EEG signal.

First of all, the method according to the invention starts from a preliminary step of having a surface EEG signal, that is acquired or has been acquired through sensing electrodes which are located on the scalp of the subject the mental workload of whom is to be estimated according to the specific directives of the 10-20 international system (e.g., see V. Jurcak et al., "10/20, 10/10, and 10/5 systems revisited: their validity as relative head-surface-based positioning systems" 2007 NeuroImage, 34 (4), 1600-1611) comprising at least one frontal channel and at least one parietal channel; in the case where the mental workload comprises or consists of a visual workload, the surface EEG signal advantageously also comprises at least one occipital channel. In the preferred embodiment, the EEG signal comprises the 6 frontal channels FPZ, F3, Fz, F4, AF3, AF4, the 4 parietal channels P3, Pz, P4, POz, and the 3 occipital channels O1, O2 and Oz, which are acquired with a sampling frequency equal to 256 Hz through respective 13 Ag/AgCl electrodes referenced to both the earlobes and grounded to the AFz electrode (according to the 10-20 international system). However, it must be noted that other embodiments of the method according to the invention may be based on surface EEG signals acquired through sensing electrodes located on the scalp differently from the 10-20 international system, but in any case located to acquire the signals of the frontal lobe and/or del parietal lobe and/or del occipital lobe of the subject's brain, still remaining within the scope of protection of the present invention.

Moreover, in the preliminary step of the preferred embodiment of the method according to the invention the electrooculogram (EOG) signal, acquired simultaneously with the EEG signal through a respective bipolar electrode located over the left eye, in order to collect the eyes movements, mainly the eye blinks, of the subjects during the execution of the task, is also available. In particular, the EEG signal (as well as the possible EOG signal) may be acquired simultaneously with the execution of the method according to the invention or it may be a recorded signal on which the method is executed, whereby the acquisition step is not essential for the method according to the invention. The preliminary step is represented by block 100 in Figure 1.

Afterwards, the EEG signal is filtered through at least one band pass filter in the frequency range from 0,1 Hz to 30 Hz, optionally from 1 Hz to 25 Hz, more optionally from 2 Hz to 20 Hz, still more optionally from 3 Hz to 15 Hz, even more optionally from 4 Hz to 12 Hz; such filtering step is represented by block 110. Optionally, said at least one band pass filter is a fourth-order Butterworth filter; the specific frequency range and the type of said at least one band pass filter may be also modified, still remaining within the scope of protection of the present invention. The filtering step 110 is not essential for the method according to the invention.

Then, the preferred embodiment of the method according to the invention, wherein step 100 also provides the EOG signal, comprises a step of removal of the artefacts wherein the eye movement contribution is removed from the EEG signal by means of the EOG signal, which step is represented by block 120; this is advantageous since the eye movement contribution could affect the theta EEG band of the EEG signal. Optionally, the removal of the eye movement contribution from the EEG signal may be carried out through the algorithm of Gratton and Coles (see Gratton et al. in "A new method for off-line removal of ocular artifact", 1983 Electroencephalogr. Clin. Neurophysiol. 55, 468-484). In artefact removal step 120, other specific algorithms (in combination with or alternatively to the removal of the eye movement contribution) of removal of different sources of artefact may be also applied, such as for instance the subject's movements during task performance or saturation of the EEG signal due to cable movements.

The EEG signal deprived of artefacts is then segmented into a plurality of epochs having a time length *Epoch*

length, shifted from each other by a time distance Shift. The time length Epoch length and the time distance Shift are selected on the basis of the duration of the task Task duration during which the mental workload estimation is carried out so that a number of observations is available for training the algorithm asSWLDA, executed by the method according to the invention later, that is larger than the number of variables. Moreover, segmentation of the EEG signal (deprived of artefacts) into epochs is selected so as to have a condition of stationarity of the EEG signal (as disclosed by R. Elul in "Gaussian behavior of the electroencephalogram: changes during performance of mental task", 1969 Science 164, 328-331), in order to proceed with the subsequent significant spectral analysis of the EEG signal. In particular, on the basis of such criteria, which will be illustrated in detail later, the preferred embodiment of the method according to the invention, wherein Task duration is equal to 2,5 minutes (i.e. to 150 seconds), it follows that Epoch length is equal to 2 seconds and Shift is equal to 0,125 seconds; obviously, such specific values are not essential for the invention. Such segmentation step is not represented by a specific block in Figure 1: it is represented by the dashed windows 115 partially overlapping each other inside block 110 and by the segments 125 of the EEG signal deprived of artefacts inside block 120.

Afterwards, the preferred embodiment of the method according to the invention comprises a step of calculation of the power spectral density (PSD) for each epoch of the EEG signal (deprived of artefacts) of each electrode, whereby PSD matrix is calculated for each time distance Shift (equal to 0,125 secondi), taking into consideration only the frequency bands involved in the mental workload estimation, namely the theta band on the frontal and occipital channels and the alpha band on the parietal channels; in other embodiments, wherein the EEG signal only comprises at least one frontal channel and at least one parietal channel, the theta band on said at least one frontal channel and the alpha band on said at least one parietal channel are taken into consideration. Such step is represented in Figure 1 by block 130. The longer the epoch segment duration (i.e. the time length Epoch length), the higher the frequency resolution of the PSD is, whereby the time length Epoch length should be ideally infinite; differently, each segment has limited duration in time, equal to 2 seconds. Therefore, as disclosed by F.J. Harris in "On the use of windows for harmonic analysis with the discrete Fourier transform", 1978 Proceedings of the IEEE 66: 51, it is advantageous to calculate the PSD by using a Hanning window of the same time length Epoch length of the considered epoch (i.e. equal to 2 secondi, that means 0,5 Hz of frequency resolution), so that the application of a Hanning window helps to smooth the contribution of the signal in proximity of the ends of the segment (i.e. of the epoch), i.e. the error due to the fact that the epoch has limited duration, thus improving the accuracy of the PSD estimation. In particular, the Hanning window has the maximum time length of the epochs, equal to Epoch length, so as not to impair the frequency resolution; should the time length for the Hanning window be reduced (and this is in any case applicable in other embodiments of the method according to the invention), then the frequency resolution would drop accordingly (e.g., if it would be reduced down to 1 second, the frequency resolution would be equal to 1 Hz, whereas if it would be reduced down to 0,5 second, the frequency resolution would be equal to 2 Hz). Alternatively, it is possible to use a different windowing, by way of example a raised-cosine window. However, it must be noted that other embodiments of the method according to the invention may calculate the PSD without using any algorithm of correction of the error due to the fact that the epoch has limited duration, and consequently without using either a Hanning window or a raised-cosine window.

Then, the preferred embodiment of the method according to the invention comprises a step of definition of the EEG frequency bands of interest for the subject, the mental workload of whom is to be estimated, through the estimation of the so-called Individual Alpha Frequency (IAF) value, according to what disclosed by W. Klimesch in "EEG alpha and theta oscillations reflect cognitive and memory performance: a review and analysis", 1999 Brain Res. Rev. 29, 169-195; such step is represented in Figure 1 by block 140. In particular, in order to have a precise estimation of the alpha peak and, hence, of the IAF value, the subject the mental workload of whom is to be estimated is asked to keep his/her own eyes closed for one minute. Afterwards, this condition is used for the IAF estimation and for the definition of the EEG bands. In this way, a spectral feature matrix is obtained in the EEG frequency bands directly correlated to the mental workload, wherein such matrix has dimensions equal to the number of EEG channels multiplied by the number of the frequency "samples" considered for the respective EEG channels, where such frequency samples may comprise a number #Theta frequency theta band samples and/or a number #Alpha frequency alpha band samples.

Come stated above, the preferred embodiment of the method according to the invention considers as variables for the classifier only the theta band (IAF-6: IAF-2) (according to the definition given by W. Klimesch, cited above, known to those skilled in the art) on the 6 frontal channels FPZ, F3, Fz, F4, AF3, and AF4 (whereby the number #Frontal sites of the frontal channels is 6) and on the 3 occipital channels O1, O2 and Oz (whereby the number #Occipital sites of the occipital channels is 3) and only the alpha band (IAF -2: IAF + 2) (still according to the definition given by W. Klimesch, cited above, known to those skilled in the art) on the 4 parietal channels P3, Pz, P4, and POz (whereby the number #Parietal sites of the parietal channels is 4).

In the preferred embodiment of the method according to the invention, it follows that:

-    the number #Observation of observations for training the innovative asSWLDA algorithm is equal to

$$\#Observation^\frown = \frac{Task\ duration - Epoch\ length}{Shift} = 1184$$

where *Task duration* is equal to 150 seconds, *Epoch length* is equal to 2 seconds, and *Shift* is equal to 0,125 seconds;
- the number *#Variables* of variables is equal to

$$\#Variables = (\#Frontal\ sites + \#Occipital\ sites) \cdot \#Theta\ frequency\ samples +$$
$$\#Parietal\ sites \cdot \#Alpha\ frequency\ samples = 104$$

where

$$\#Theta\ frequency\ samples = (\mathrm{IAF} - 6 : \mathrm{IAF} - 2) \cdot \frac{1}{Epoch\ length} = 8$$

and

$$\#Alpha\ frequency\ samples = (\mathrm{IAF} - 2 : \mathrm{IAF} + 2) \cdot \frac{1}{Epoch\ length} = 8$$

It must be understood that for the embodiments of the method according to the invention wherein the EEG signal only comprises at least one frontal channel and at least one parietal channel, the number *#Variables* of variables is equal to

$$\#Variables = \#Frontal\ sites \cdot \#Theta\ frequency\ samples +$$
$$\#Parietal\ sites \cdot \#Alpha\ frequency\ samples$$

Moreover, other embodiments of the method according to the invention may refer to definitions of the theta and alfa bands and related frequency samples different from the one by W. Klimesch (cited above), still remaining within the scope of protection of the present invention.

Afterwards, the preferred embodiment of the method according to the invention comprises a step of execution of the innovative asSWLDA technique, i.e. the SWLDA algorithm with automatic stop, that is represented in Figure 1 by block 150. In particular, the entry significance threshold $\alpha_{\mathrm{ENTER}}$ and the removal significance threshold $\alpha_{\mathrm{REMOVE}}$ are set as in the classical SWLDA algorithm, whereby:

$$\alpha_{\mathrm{ENTER}} = 0{,}05$$

and

$$\alpha_{\mathrm{REMOVE}} = 0{,}1$$

In this regard, as already stated for the classical SWLDA algorithm, it is also possible to use other values for $\alpha_{\mathrm{ENTER}}$ and $\alpha_{\mathrm{REMOVE}}$, whereby such values are not an essential feature for the invention. Optionally, to obtain a better statistical significance, the entry significance threshold $\alpha_{\mathrm{ENTER}}$ is not higher than 0,05 and the removal significance threshold $\alpha_{\mathrm{REMOVE}}$ is not higher than 0,1 whereby:

$$\alpha_{\mathrm{ENTER}} \leq 0{,}05$$

and

$$\alpha_{REMOVE} \leq 0,1$$

Differently, the value of the maximum number $Iteration_{MAX}$ of iterations is selected so that the optimal number $\#Features_{OPTIMUM}$ of features inserted in the model is chosen so that it is much larger than the number $\#Features_{UNDERFITTING}$ of features which would cause the underfitting phenomenon and much lower than the number $\#Features_{OVERFITTING}$ of features which would cause the overfitting phenomenon, whereby:

$$\#Features_{UNDERFITTING} \ll \#Features_{OPTIMUM} \ll \#Features_{OVERFITTING}$$

In order to make the classifier be capable to automatically find the best $Iteration_{MAX}$ parameter, the method takes into account the p value (in the following also indicated as pModel) of the whole model, which value is available at the output of the standard SWLDA implementation and that gives information about the global significance of the model at the iteration j-th iteration. In particular, the pModel differs from the value $pval_{ij}$ used in the standard SWLDA because the latter represents the p value of the i-th feature at the j-th iteration, i.e. it refers to a statistics calculated on the single feature (conditioned to the features already present in the model), while the pModel represents an index of statistical significance of the whole model, with all the features which have been inserted in the same model.

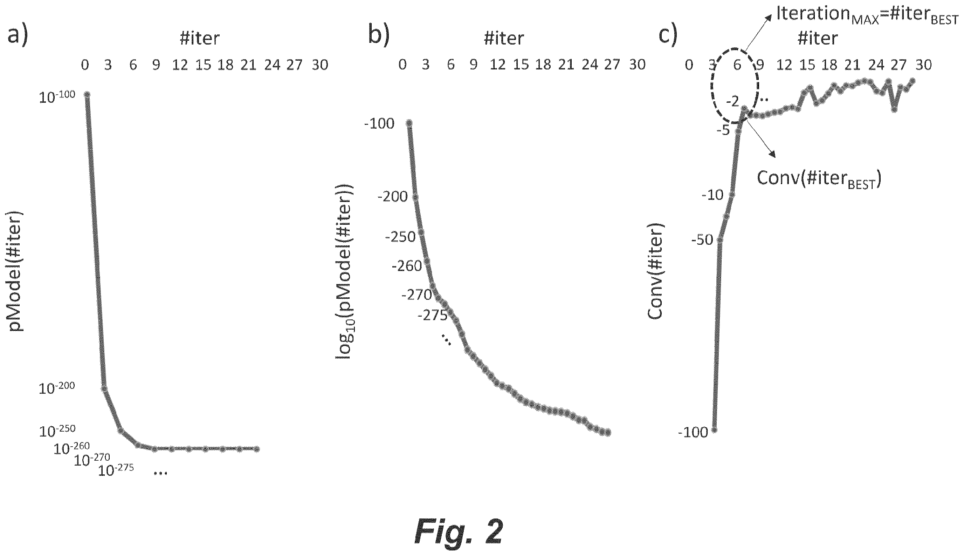

The inventors have realized that the more the number of iterations increases (i.e. the more features are added to the model), the more the pModel decreases (tending to zero) with a decreasing exponential shape. In step 150, firs of all the method according to the invention collects all the values pModel for all the performed iterations in a vector; in this regard, Figure 2a shows the values pModel for all the iterations *(pModel(#iter))* (where *#iter* is the iteration ordinal) of an exemplary execution of the preferred embodiment of the method according to the invention. Then, the method calculates a vector equal to the base 10 logarithm $(log_{10})$ of the vector of the values pModel; Figure 2b shows the logarithm $log_{10}(pModel(\#iter))$ of the values pModel shown in Figure 2a. Afterwards, the method calculates a vector the elements of which are the first order differences between adjacent elements of the vector of the base 10 logarithm of the adjacent values pModel, which vector is also indicated in the following as convergence function *Conv*(#iter), as follows:

$$Conv(\#iter) = log_{10}(pModel(\#iter + 1)) - log_{10}(pModel(\#iter))$$

The convergence function *Conv*(#iter) gives information on the differences between the orders of magnitude of the values pModel (since the base 10 logarithm gives information on the order of magnitude of the values pModel). Figure 2c shows the convergence function *Conv*(#iter) of the logarithm $log_{10}(pModel(\#iter))$ shown in Figure 2b.

On the basis of the convergence function *Conv*(#iter), the method according to the invention identifies the best value $Iteration_{MAX-OPTIMUM}$ of the parameter $Iteration_{MAX}$ as the number of iterations corresponding to the value #iter at which the convergence function Conv(#iter) assumes the lowest distance from the origin (0,0) (i.e. it assumes minimum norm from the point (0,0)), incremented by one (since the preferred embodiment is operating on the first order differences), whereby:

$$Iteration_{MAX\_OPTIMUM} = \#iter_{BEST} + 1$$

where

$$\|Conv(\#iter_{BEST})\| = min\|Conv(\#iter)\|$$

In fact, the inventors have realized that the best condition would be having the least possible features and at the same time the convergence of the model, according to the following formula:

$$log_{10}(pModel(\#iter + 1)) - log_{10}\big(pModel(\#iter)\big) = 0$$

In particular, in Figure 2c it is highlighted that $\#iter_{BEST}$ is equal to 6, since for such value #iter the convergence function Conv(#iter) assumes the lowest distance from the origin (0,0), equal to:

$$\|Conv(\#iter_{BEST})\| = min\|Conv(\#iter)\| = \sqrt{(-2-0)^2 + (6-0)^2} = 2\sqrt{10}$$

The mental workload estimation is then performed by calculating a mental workload index $W_{EEG}$ on the basis of the subject's EEG activity.

In particular, in the preferred embodiment of the method according to the invention, the subject the mental workload of whom is to be estimated performs a task under three different difficulty levels (indicated as Easy, Medium, Hard), and step 150 is performed wherein a three-class automatic-stop SWLDA (asSWLDA) regression is used to select within a training dataset the most relevant EEG spectral features of the EEG signal to discriminate the mental workload within the three conditions (i.e. within the three classes Easy, Medium, Hard).

Afterwards, the preferred embodiment of the method according to the invention comprises a step of calculation of a linear discriminant function *ytest(t)* for the PSD of each epoch, by using the coefficients, namely the weights $wi_{train}$ and the intercept *btrain,* returned by the asSWLDA regression, as follows:

$$y_{test}(t) = \sum_i w_{i\,train} \cdot f_{i\,test}(t) + b_{train}$$

where $fi_{test}(t)$ represents the PSD matrix of the analysis dataset (testing dataset), i.e. of all the epochs into which the EEG signal has been segmented, at the sampling time t relating to the i-th feature. In other words, the coefficients (i.e. the weights $wi_{train}$ and the intercept $b_{train}$) derived from the training dataset are used and combined with the PSD epochs of the testing dataset for calculating the linear discriminant function *ytest(t).* Such step is represented in Figure 1 by block 160.

Finally, the preferred embodiment of the method according to the invention comprises a step represented in Figure 1 by block 170, where a simple moving average (i.e. not weighted) of 8 seconds (8MA) is applied to the function *ytest(t)* in orderto smooth the same function *ytest(t)* by reducing the variance of the measure, obtaining the index $W_{EEG}$ of mental workload based on the EEG signal, as follows:

$$W_{EEG} = 8MA\left(y_{test}(t)\right)$$

It must be noted that other embodiments of the method according to the invention may have a step 170 of smoothing of the function *ytest(t)* wherein a simple moving average of duration different from 8 seconds, and/or a weighted moving average and/or a different algorithm of smoothing is applied to reduce the variability of the function *ytest(t).* Also, further embodiments of the method according to the invention may also not comprise any smoothing step 170, that is not an essential feature for the invention, whereby, in such case, the mental workload estimation is equal to the same function *ytest(t).*

The apparatus configured to execute the method according to the invention comprises a processing unit configured to execute the method according to the invention and to display the results thereof, in particular the function *ytest(t)* and/or the mental workload index $W_{EEG}$, on a display, that is advantageously part of the apparatus according to the invention. The results of the method may be also displayed "on-line", since all the steps of the method according to the invention (for instance including the artefact removal) do not need a posteriori information. Optionally, the apparatus comprises two or more electrodes and the related electronic circuitry (e.g. at least one signal amplifier and at least one sampling device) which are connected, preferably through cables, to the processing unit and configured to be located on a scalp and to acquire surface EEG signals (comprising at least one frontal channel and at least one parietal channel). Advantageously, the processing unit is provided with an input/output interface device, for reading data stored in an external memory medium or received from a network to which the input/output interface device connects, and with a memory configured to store the data read and/or the data acquired by the electrodes.

Also, the method according to the invention may be implemented through at least one computer program, specifically adapted to execute, when launched on a processor, the method for estimating a mental state of a subject during performance of a task according to the invention.

Furthermore, such at least one computer program may be stored in a computer-readable memory medium.

The inventors have conducted some tests based on the preferred embodiment of the method according to the invention, comparing the innovative asSWLDA technique on which it is based with a standard implementation of the SWLDA technique. In particular, the method according to the invention has been tested with ten healthy subjects while performing the MATB task (see Comstock, 1994, cited above) simulating the cockpit of an airplane, wherein the performed tasks have been executed under three different difficulty levels (Easy, Medium, Hard) resembling different possible flight scenarios (cruise phase, maintaining flight level, and emergency condition). The subjects have been trained to use the MATB before starting with the tests, and throughout the experimental sessions they maintained the previously acquired skills.

The AUC *(Area Under Curve)* regression performance analyses, carried out according the technique disclosed by D. Bamber in "The area above the ordinal dominance graph and the area below the receiver operating characteristic graph", 1975 Journal of Mathematical Psychology 12, 387-415, and the distributions of the mental workload index $W_{EEG}$ have shown that when the asSWLDA is trained with the features correlated to the mental workload (i.e. to the EEG theta frontal and occipital and alpha parietal rhythms), it is possible to render the workload measure stable over subsequent days (at least up to one week) without significant decreases between the cross-validations in the same day (i.e. wherein the test data are acquired in the same day of the training dataset), short-term cross-validations (wherein the test data are acquired with one, indifferently previously or next, day of distance with respect to the day of acquisition of the training dataset) and medium term cross-validations (wherein the test data are acquired with 7 or 8 days of distance with respect to the day of acquisition of the training dataset). This means that even after one week, it is not required to recalibrate the method according to the invention with new subject's EEG data. On the contrary, the standard implementation of SWLDA is not capable to select features so that the classification remains stable over different days, making the classification too specific and related to the training dataset of the same day.

Moreover, the tests conducted by the inventors have shown that the method according to the invention is capable to significantly differentiate the mental workload in all the three experimental conditions (Easy, Medium, Hard). In this regard, it is important to point out that the neurophysiological measure provided by the method according to the invention has a resolution much higher than the NASA-TLX subjective evaluation (disclosed by Hart, 1988, cited above).

The preferred embodiments of this invention have been described and a number of variations have been suggested hereinbefore, but it should be understood that those skilled in the art can make other variations and changes without so departing from the scope of protection thereof, as defined by the attached claims.

## Claims

1. Method for estimating a mental state of a subject during performance of a task, comprising the following steps:

   A. having (100) a surface EEG electroencephalographic signal comprising one or more frontal acquisition channels and one or more parietal acquisition channels;
   B. segmenting said surface EEG signal into a plurality of epochs having a time length *Epoch length,* shifted from each other by a time distance *Shift;*
   C. calculating (130) a power spectral density for each epoch of said plurality of epochs, wherein said power spectral density is calculated in theta band for said one or more frontal channels and in alpha band for said one or more parietal channels of the surface EEG signal;
   D. defining (140) frequency bands of interest of the surface EEG signal through an estimation of the Individual Alpha Frequency (IAF), whereby a spectral features matrix is obtained in said frequency bands of interest having size equal to the number of said one or more acquisition channels multiplied by the number of frequency samples, where such frequency samples comprise a number *#Theta frequency samples* of theta band samples for said one or more frontal channels of the surface EEG signal and a number *#Alpha frequency samples* of alpha band samples for said one or more parietal channels of the surface EEG signal; **characterised in that** the method further comprises the steps of:

      E. executing (150) a stepwise linear discriminant analysis SWLDA with automatic stop, trained on a training dataset, that provides weights $wi_{train}$ and intercept $b_{train}$, through the following substeps:

         E1. setting an entry significance threshold $\alpha_{ENTER}$ and a removal significance threshold $\alpha_{REMOVE}$,
         E2. collecting the values pModel of the whole model for all the iterations carried out by the stepwise linear discriminant analysis SWLDA in a first vector,
         E3. calculating a second vector the elements of which are equal to the base 10 logarithm of the elements of the first vector, whereby the elements of the second vector are base 10 logarithms of the values pModel for all the iterations carried out by the stepwise linear discriminant analysis SWLDA,
         E4. calculating a third vector *Conv*(#iter) the elements of which are the first order differences between adjacent elements of the second vector, as follows:

$$Conv(\#iter) = log_{10}(pModel(\#iter + 1)) - log_{10}(pModel(\#iter))$$

E5. determining as best value $Iteration_{MAX-OPTIMUM}$ of the number $Iteration_{MAX}$ of iterations of the stepwise linear discriminant analysis SWLDA the number of iterations corresponding to the value #iter of the iteration at which the third vector Conv(#iter) assumes minimum norm from point (0,0), incremented by one, whereby:

$$Iteration_{MAX\_OPTIMUM} = \#iter_{BEST} + 1$$

where

$$\|Conv(\#iter_{BEST})\| = min\|Conv(\#iter)\| \; ;$$

F. calculating (160) a linear discriminant function *ytest(t)* for the power spectral density for each epoch of said plurality of epochs by using the weights $wi_{train}$ and the intercept $b_{train}$ provided by the stepwise linear discriminant analysis SWLDA, as follows:

$$y_{test}(t) = \sum_{i} w_{i\ train} \cdot f_{i\ test}(t) + b_{train}$$

where $fi_{test}(t)$ is the matrix of the power spectral densities of a testing dataset at the sampling time t related to a i-th spectral feature,

wherein an estimation of said mental state is function of the linear discriminant function *ytest(t),* and wherein the time length *Epoch length* and the time distance *Shift* are selected on the basis of the task duration *Task duration* so that the observation number *#Observation* for training the stepwise linear discriminant analysis SWLDA is larger than the variable number *#Variables,* where

$$\#Observation. \ = \frac{Task\ duration - Epoch\ length}{Shift}$$

and

$$\#Variables = \#Frontal\ sites \cdot \#Theta\ frequency\ samples +$$
$$' \#Parietal\ sites \cdot \#Alpha\ frequency\ samples$$

wherein *#Frontal sites* is the number of said one or more frontal channels and *#Paurietul sites* is the number of said one or more parietal channels.

2. Method according to claim 1, **characterised in that** the surface EEG electroencephalographic signal available in step A further comprises one or more occipital acquisition channels, in step C (130) said power spectral density is calculated in the theta band for said one or more occipital channels of the surface EEG signal, in step D such frequency samples comprise a number *#Theta frequency samples* of theta band samples for said one or more frontal channels and said one or more occipital channels of the surface EEG signal, wherein the variable number *#Variables* is incremented by the amount

$$\#Occipital\ sites \cdot \#Theta\ frequency\ samples$$

wherein *#Occipital sites* is the number of said one or more occipital channels, whereby the variable number *#Variables* is equal to

$$\#Variables = (\#Frontal\ sites + \#Occipital\ sites) \cdot \#Theta\ frequency\ samples +$$

$$\#Parietal\ \ sites \cdot \#Alpha\ frequency\ samples$$

3. Method according to claim 2, **characterised in that** said EEG signal is acquired or has been acquired through sensing electrodes located on a scalp of the subject a mental state of whom is to be estimated according to the 10-20 international system and optionally comprises the six frontal channels FPZ, F3, Fz, F4, AF3, AF4, the four parietal channels P3, Pz, P4, POz, and the three occipital channels O1, O2 and Oz, whereby in step C said power spectral density is calculated in the theta band for the six frontal channels FPZ, F3, Fz, F4, AF3, AF4, and for the three occipital channels O1, O2 and Oz and in the alpha band for the four parietal channels P3, Pz, P4, POz, and wherein

$$\#Theta\ frequency\ bins = (\text{IAF} - 6 : \text{IAF} - 2) \cdot \frac{1}{Epoch\ length}$$

and

$$\#Alpha\ frequency\ bins = (\text{IAF} - 2 : \text{IAF} + 2) \cdot \frac{1}{Epoch\ length}$$

4. Method according to any one of the preceding claims, **characterised in that** it comprises, after step A and before step C, the following additional step:

G. filtering (110) said EEG signal through at least one band pass filter, optionally in the frequency range from 0,1 Hz to 30 Hz, more optionally from 1 Hz to 25 Hz, still more optionally from 2 Hz to 20 Hz, even more optionally from 3 Hz to 15 Hz, still even more optionally from 4 Hz to 12 Hz, even still more optionally through at least one fourth-order Butterworth filter.

5. Method according to any one of the preceding claims, **characterised in that** it comprises, after step A and before step C, the following additional step:

H. removing one or more artefacts from the EEG signal,

wherein said one or more artefacts are selected from the group comprising artefacts due to ocular movements, artefacts due to movements of the subject a mental state of whom is to be estimated, artefacts due to saturation of the EEG signal.

6. Method according to claim 5, **characterised in that** step H removes artefacts due to ocular movements from the EEG signal through an EOG electrooculography signal that is available in step A (100), wherein the EOG signal is acquired or has been acquired simultaneously with the EEG signal.

7. Method according to any one of the preceding claims, **characterised in that** step C (130) calculates said power spectral density by using a Hanning window, optionally having time length equal to *Epoch length,* or a raised-cosine window.

8. Method according to any one of the preceding claims, **characterised in that** said estimation of said mental state is the identity function of the linear discriminant function *ytest(t)*, whereby it coincides with the linear discriminant function *ytest(t).*

9. Method according to any one of claims 1 to 7, **characterised in that** said estimation of said mental state is an index $W_{EEG}$ equal to a moving average of the linear discriminant function *ytest(t).*

10. Method according to claim 9, **characterised in that** said index $W_{EEG}$ is equal to a simple moving average, optionally of duration equal to 4 times the time length *Epoch length* of the epochs, of the linear discriminant function *ytest(t).*

11. Method according to claim 9, **characterised in that** said index $W_{EEG}$ is equal to a weighted moving average of the linear discriminant function *ytest(t)*.

12. Apparatus for estimating a mental state of a subject during performance of a task, **characterised in that** it comprises a processing unit configured to execute the method for estimating a mental state of a subject during performance of a task according to any one of claims 1 to 11.

13. Apparatus according to claim 12, **characterised in that** it further comprises one or more electrodes configured to be connected, optionally through cables, to the processing unit and configured to be located on a scalp and to acquire EEG signals.

14. Computer program **characterised in that** it is adapted to execute the method for estimating a mental state of a subject during performance of a task according to any one of claims 1 to 11.

15. Computer-readable memory medium **characterised in that** it stores the computer program according to claim 14.

**Patentansprüche**

1. Verfahren zum Schätzen eines mentalen Zustandes eines Subjekts während der Ausführung einer Aufgabe, umfassend die folgenden Schritte:

   A. Verwenden (100) eines Oberflächen-EEG-Elektroenzephalographischen Signals, das einen oder mehrere frontale Aufnahmekanäle und einen oder mehrere parietale Aufnahmekanäle umfasst;
   B. Segmentieren des Oberflächen-EEG-Signals in eine Vielzahl von Epochen, die eine zeitliche Länge *Epoch length* aufweisen, welche gegeneinander verschoben sind um eine Zeitverschiebung *Shift;*
   C. Berechnen (130) einer spektralen Leistungsdichte für jede Epoche der Vielzahl von Epochen, wobei die spektrale Leistungsdichte im Theta-Band für den einen oder die mehreren frontalen Kanäle und im Alpha-Band für den einen oder die mehreren parietalen Kanäle des Oberflächen-EEG-Signals berechnet wird;
   D. Definieren (140) interessierender Frequenzbänder des Oberflächen-EEG-Signals durch eine Schätzung der individuellen Alpha-Frequenz (IAF), wodurch eine Spektralmerkmalsmatrix in den Frequenzbändern von Interesse mit einer Größe erhalten wird, die gleich der Anzahl der genannten Erfassungskanäle multipliziert mit der Anzahl von Frequenzabtastwerten ist, wobei solche Frequenzabtastwerte eine Anzahl *#Theta frequency samples* von Theta-Bandabtastwerten für den einen oder die mehreren frontalen Kanäle des Oberflächen-EEG-Signals und eine Anzahl *#Alpha frequency samples* von Alphabandabtastwerten der eine oder mehrere parietale Kanäle des Oberflächen-EEG-Signals umfassen;

   **dadurch gekennzeichnet, dass** das Verfahren zudem die Schritte umfasst:

   E. Ausführen (150) einer schrittweisen linearen Diskriminanzanalyse SWLDA mit automatischem Stopp, trainiert auf einem Trainingsdatensatz, der Gewichte $wi_{train}$ und einen Schnittpunkt $bt_{rain}$ durch die folgenden Unterschritte bereitstellt:

   E1 setzen eine Eintrittsbedeutungsschwelle $\alpha_{ENTER}$ und eine Entfernungssignifikanzschwelle $\alpha_{REMOVE}$,
   E2 Sammeln der Werte pModel des gesamten Modells für alle Iterationen, die durch die schrittweise lineare Diskriminanzanalyse SWLDA in einem ersten Vektor ausgeführt werden, E3 berechnet einen zweiten Vektors, dessen Elemente gleich dem Logarithmus der Basis 10 der Elemente des ersten Vektors sind, wobei die Elemente des zweiten Vektors Basis-10-Logarithmen der Werte pModel für alle Iterationen sind, die durch Ausführen der schrittweise lineare Diskriminanten Analyse SWLDA erhalten werden,
   E4 Berechnen eines dritten Vektors *Conv (#iter),* dessen Elemente die Differenzen erster Ordnung zwischen benachbarten Elementen des zweiten Vektors sind, wie folgt:

   $$Conv(\#iter) = log_{10}(pModel(\#iter + 1)) - 1og_{10}(pModel(\#iter))$$

   E5. Bestimmen der Anzahl von Iterationen als besten Wert *Iteration$_{MAX\_OPTIMUM}$* der Anzahl *Iteration$_{MAx}$* von Iterationen der schrittweisen linearen Diskriminanzanalyse SWLDA entsprechend dem Wert *#iter* der Iteration, bei der der dritte Vektor *Conv (#iter)* gegenüber Punkt (0, 0) eine minimale Norm aufweist, inkre-

mentiert um eins, wobei:

$$Iteration_{MAx\ OPTIMUM} = \#iter_{BEST} + 1$$

mit

$$|| Conv(\#iter_{BEST}) || = min\ ||Conv\ (\#iter) ||\ ;$$

F. Berechnen (160) einer linearen Diskriminanzfunktion *ytest (t)* für die spektrale Leistungsdichte für jede Epoche der mehreren Epochen unter Verwendung der Gewichtungen $wi_{train}$ und des Wertes $b_{train}$, die durch die schrittweise lineare Diskriminanzanalyse SWLDA bereitgestellt werden, wie folgt:

$$Ytest\ (t) = \quad \Sigma_i\ W_{i\ train}\ \ f_{i\ test}\ (t) + b_{train}$$

wobei $f_{itest}$ (t) die Matrix der spektralen Leistungsdichten eines Testdatensatzes zum Abtastzeitpunkt t bezogen auf ein i-tes spektrales Merkmal ist, wobei eine Schätzung des mentalen Zustandes eine Funktion der linearen Diskriminanzfunktion *ytest (t)* ist, und wobei die Zeitdauer *Epoch length* und die Zeitentfernung *Shift* auf der Basis der Taskdauer *Task duration* ausgewählt werden, so dass die Beobachtungsnummer *#Observation* zum Trainieren der schrittweisen linearen Diskriminanzanalyse SWLDA größer ist als die variable Anzahl *#Variables,* wobei

$$\#Observation: = (Task\ duration - Epoch\ length)\ /\ Shift$$

und

$$\#Variables = \#Frontal\ sites \bullet \#Theta\ frequency\ samples + '\ \#Parietal\ sites \bullet \#Alpha\ frequency\ samples$$

wobei *#Frontal sites* die Nummer des einen oder der mehreren frontalen Kanäle und *#Parietal Sites* die Anzahl des einen oder der mehreren parietalen Kanäle ist.

2. Verfahren nach Anspruch 1, **dadurch gekennzeichnet, dass** das in Schritt A verfügbare Oberflächen-EEG-Elektroenzephalogramm weiterhin einen oder mehrere Okzipitalakquisitionskanäle umfasst, wobei in Schritt C (130) die Leistungsspektraldichte im Theta-Band für den einen oder die mehreren Okzipitalkanäle des Oberflächen-EEG-Signals berechnet wird, wobei in Schritt D solche Frequenzabtastungen eine Anzahl von *#Theta frequency samples* der t-Band-Abtastwerte für den einen oder die mehreren frontalen Kanäle und den einen oder die mehreren okzipitalen Kanäle des Oberflächen-EEG-Signals umfassen, wobei die variable Anzahl *#Variables* um den Betrag

$$\#Occipital\ sites \bullet \#Theta\ frequency\ samples$$

erhöht ist, wobei *#Occipital sites* die Anzahl der zuvor genannten ein oder mehreren Okzipitalkanäle ist, wobei die Variablennummer *#Variables* gleich

$$\#Variables = (\#Frontal\ sites + \#Occipital\ sites) \bullet \#Theta\ frequency\ samples + \#Parietal\ sites \bullet \#\ Alpha\ frequency\ samples$$

ist.

3. Verfahren nach Anspruch 2, **dadurch gekennzeichnet, dass** das EEG-Signal durch auf einer Kopfhaut des Subjekts befindliche Erfassungselektroden erfasst oder gewonnen wurde, von denen ein mentaler Zustand gemäß dem internationalen System 10-20 abzuschätzen ist und optional die sechs frontalen Kanäle FPZ, F3, Fz, F4, AF3, AF4, die vier parietalen Kanäle P3, Pz, P4, POz und die drei okzipitalen Kanäle 01, 02 und Oz umfasst, wobei in Schritt C die spektrale Leistungsdichte für das Thetaband für die sechs frontalen Kanäle FPZ, F3, Fz, F4, AF3, AF4 und für die drei occipitalen Kanäle 01, 02 und Oz und im Alphaband für die vier parietalen Kanäle P3, Pz, P4, POz berechnet wird und worin

$$\#Theta\ frequency\ bins = (IAF - 6: IAF - 2) \bullet 1/(Epoch\ length)$$

und

$$\#Alpha\ frequency\ bins = (IAF - 2: IAF + 2) \bullet 1/(Epoch\ length).$$

4. Verfahren nach einem der vorhergehenden Ansprüche, **dadurch gekennzeichnet, dass** dieses nach Schritt A und vor Schritt C folgenden zusätzlichen Schritt umfasst:

G. Filtern (110) des EEG-Signals durch mindestens einen Bandpassfilter, optional in dem Frequenzbereich von 0,1 Hz bis 30 Hz, vorzugsweise von 1 Hz bis 25 Hz, weiter bevorzugt von 2 Hz bis 20 Hz, weiter bevorzugt von 3 Hz bis 15 Hz, weiter bevorzugt von 4 Hz bis 12 Hz, weiter bevorzugt durch mindestens einen Butterworth-Filter vierter Ordnung.

5. Verfahren nach einem der vorhergehenden Ansprüche, **dadurch gekennzeichnet, dass** dieses nach Schritt A und vor Schritt C folgenden zusätzlichen Schritt umfasst:

H. Entfernen eines oder mehrerer Artefakte aus dem EEG-Signal, wobei das eine oder die mehreren Artefakte ausgewählt sind aus der Gruppe bestehend aus Artefakten aufgrund von Augenbewegungen, Artefakten aufgrund von Bewegungen des Subjekts, deren mentaler Zustand geschätzt werden soll, Artefakten aufgrund der Sättigung des EEG-Signals.

6. Verfahren nach Anspruch 5, **dadurch gekennzeichnet, dass** Schritt H Artefakte aufgrund von Augenbewegungen aus dem EEG-Signal durch ein in Schritt A (100) verfügbares EOG Elektrookulographie-Signal entfernt, wobei das EOG-Signal gleichzeitig mit dem EEG-Signal erfasst wird oder erfasst wurde.

7. Verfahren nach einem der vorhergehenden Ansprüche, **dadurch gekennzeichnet, dass** Schritt C (130) die Leistungsspektraldichte unter Verwendung eines Hanning-Fensters berechnet, das optional eine Zeitlänge gleich der Länge *Epoch length* oder ein Wurzel-Kosinus Fenster aufweist.

8. Verfahren nach einem der vorhergehenden Ansprüche, **dadurch gekennzeichnet, dass** die Schätzung des mentalen Zustandes die Identitätsfunktion des linearen Diskriminanzfunktionstests (t) ist, wobei diese mit der linearen Diskriminanzfunktion *ytest (t)* übereinstimmt.

9. Verfahren nach einem der Ansprüche 1 bis 7, **dadurch gekennzeichnet, dass** die Schätzung des mentalen Zustands ein Index $W_{EEG}$ ist, der gleich einem gleitenden Durchschnitt der linearen Diskriminanzfunktion *ytest (t)* ist.

10. Verfahren nach Anspruch 9, **dadurch gekennzeichnet, dass** der Index $W_{EEG}$ gleich einem einfachen gleitenden Durchschnitt ist, der optional eine Dauer hat, die gleich dem Vierfachen der *Epoch length* der Epochen der linearen Diskriminanzfunktion *ytest (t)* ist.

11. Verfahren nach Anspruch 9, **dadurch gekennzeichnet, dass** der Index $W_{EEG}$ gleich einem gewichteten gleitenden Durchschnitt der linearen Diskriminanzfunktion *ytest (t)* ist.

12. Vorrichtung zum Schätzen eines mentalen Zustandes eines Subjekts während der Ausführung einer Aufgabe, **dadurch gekennzeichnet, dass** diese eine Verarbeitungseinheit umfasst, die konfiguriert ist, das Verfahren zum Schätzen eines mentalen Zustandes eines Subjekts während der Ausführung einer Aufgabe nach einem der Ansprüche 1 bis 11 auszuführen.

**13.** Vorrichtung nach Anspruch 12, **dadurch gekennzeichnet, dass** diese ferner eine oder mehrere Elektroden umfasst, die so konfiguriert sind, dass sie gegebenenfalls über Kabel mit der Verarbeitungseinheit verbunden und so konfiguriert sind, dass sie auf einer Kopfhaut angeordnet sind und EEG-Signale erhalten.

**14.** Computerprogramm, **dadurch gekennzeichnet, dass** dieses angepasst ist, um das Verfahren zum Schätzen eines mentalen Zustandes eines Subjekts während der Ausführung einer Aufgabe gemäß einem der Ansprüche 1 bis 11 auszuführen.

**15.** Computerlesbares Speichermedium, **dadurch gekennzeichnet, dass** es das Computerprogramm nach Anspruch 14 speichert.

**Revendications**

**1.** Procédé d'estimation d'un état mental d'un sujet pendant l'exécution d'une tâche, comprenant les étapes suivantes :

A. obtenir (100) un signal électro-encéphalographique EEG de surface comprenant un ou plusieurs canaux d'acquisition frontaux et un ou plusieurs canaux d'acquisition pariétaux ;
B. segmenter ledit signal EEG de surface en une pluralité d'époques ayant une durée *Epoch length,* décalées les unes des autres par une distance temporelle *Shift ;*
C. calculer (130) une densité spectrale de puissance pour chaque époque de ladite pluralité d'époques, où ladite densité spectrale de puissance est calculée dans la bande thêta pour ledit ou lesdits canaux frontaux et dans la bande alpha pour ledit ou lesdits canaux pariétaux du signal EEG de surface ;
D. définir (140) bandes de fréquences d'intérêt du signal EEG de surface par estimation de la fréquence alpha individuelle (IAF), au moyen de laquelle une matrice de caractéristiques spectrales dans lesdites bandes de fréquences d'intérêt de taille égale au nombre dudit ou desdits canaux d'acquisition multiplié par le nombre d'échantillons de fréquences est obtenue, où ces échantillons de fréquences comprennent un nombre #*Theta frequency samples* d'échantillons de bande thêta pour ledit ou lesdits canaux frontaux du signal EEG de surface et un nombre #*Alpha frequency samples* d'échantillons de bande alpha pour ledit ou lesdits canaux pariétaux du signal EEG de surface;

**caractérisé en ce que** le procédé comprend en outre les étapes suivantes :

E. exécuter (150) une analyse discriminante linéaire pas-à-pas SWLDA avec arrêt automatique, entraînée sur un jeu de données d'entraînement, qui fournit les poids $wi_{train}$ et l'ordonnée à l'origine $b_{train}$, par l'intermédiaire des sous-étapes suivantes :

E1. définir un seuil de signification d'entrée $\alpha_{ENTER}$ et un seuil de signification d'élimination $\alpha_{REMOVE}$,
E2. collecter les valeurs pModel de tout le modèle pour toutes les itérations effectuées par l'analyse discriminante linéaire pas-à-pas SWLDA dans un premier vecteur,
E3. calculer un deuxième vecteur dont les éléments sont égal au logarithme de base 10 des éléments du premier vecteur, au moyen duquel les éléments du deuxième vecteur sont des logarithmes de base 10 des valeurs pModel de toutes les itérations effectuées par l'analyse discriminante linéaire pas-à-pas SWLDA,
E4. calculer un troisième vecteur *Conv(#iter)* dont les éléments sont les différences de premier ordre entre des éléments adjacents du deuxième vecteur, comme suit:

$$Conv(\#\text{iter}) = log_{10}(pModel(\#\text{iter}+1)) - log_{10}(pModel(\#\text{iter}))$$

E5. déterminer, comme meilleure valeur *Iteration*$_{MAX\_OPTIMUM}$ du nombre Iteration$_{MAX}$ d'itérations de l'analyse discriminante linéaire pas-à-pas SWLDA, le nombre d'itérations correspondant à la valeur #iter de l'itération à laquelle le troisième vecteur *Conv*(#iter) assume la norme minimale par rapport au point (0,0), incrémenté de un, selon lequel:

$$Iteration_{MAX\_OPTIMUM} = \#iter_{BEST} + 1$$

et :

$$\|Conv(\#iter_{BEST})\| = min\|Conv(\#iter)\| \ ;$$

F. calculer (160) une fonction discriminante linéaire *ytest(t)* pour la densité spectrale de puissance de chaque époque de ladite pluralité d'époques à l'aide des poids $wi_{train}$ et de l'ordonnée à l'origine $b_{train}$ fournis par l'analyse discriminante linéaire pas-à-pas SWLDA, comme suit :

$$y_{test}(t) = \sum_i w_{i\ train} \cdot f_{i\ test}(t) + b_{train}$$

où $fi_{test}(t)$ est la matrice des densités spectrales de puissance d'un jeu de données de test au temps d'échantillonnage t associé à une i-ème caractéristique spectrale,

dans lequel une estimation dudit état mental est fonction de la fonction discriminante linéaire *ytest(t),* et dans lequel la durée *Epoch length* et la distance temporelle *Shift* sont choisies sur la base de la durée de la tâche *Task duration* de façon que le nombre d'observations *#Observation* pour l'entraînement de l'analyse discriminante linéaire pas-à-pas SWLDA soit supérieur au nombre de variables *#Variables,* où

$$\#Observation. = \frac{Task\ duration - Epoch\ length}{Shift}$$

et

$$\#Variables = \#Frontal\ sites \cdot \#Theta\ frequency\ samples +$$
$$\#Parietal\ sites \cdot \#Alpha\ frequency\ samples$$

où *#Frontal sites* est le nombre dudit ou desdits canaux frontaux et *#Parietal sites* est le nombre dudit ou desdits canaux pariétaux.

2. Procédé selon la revendication 1, **caractérisé en ce que** le signal électro-encéphalographique EEG de surface disponible à l'étape A comprend en outre un ou plusieurs canaux d'acquisition occipitaux, **en ce qu'**à l'étape C (130) ladite densité spectrale de puissance est calculée dans la bande thêta dudit ou desdits canaux occipitaux du signal EEG de surface, **en ce qu'**à l'étape D ces échantillons de fréquences comprennent un nombre *#Theta frequency samples* d'échantillons de bande thêta pour ledit ou lesdits canaux frontaux et ledit ou lesdits canaux occipitaux du signal EEG de surface, où le nombre de variables *#Variables* est incrémenté de la quantité

$$\#Occipital\ sites \cdot \#Theta\ frequency\ samples$$

où *#Occipital sites* est le nombre dudit ou desdits canaux occipitaux, par lequel le nombre de variables *#Variables* est égal à

$$\#Variables = (\#Frontal\ sites + \#Occipital\ sites) \cdot \#Theta\ frequency\ samples +$$
$$\#Parietal\ sites \cdot \#Alpha\ frequency\ samples$$

3. Procédé selon la revendication 2, **caractérisé en ce que** ledit signal EEG est acquis ou a été acquis par l'intermédiaire d'électrodes de détection placées sur le scalp du sujet dont l'état mental doit être estimé selon le système international 10-20 et comprend éventuellement les six canaux frontaux FPZ, F3, Fz, F4, AF3, AF4, les quatre canaux pariétaux P3, Pz, P4, POz, et les trois canaux occipitaux O1, O2 et Oz, par lequel à l'étape C ladite densité spectrale de puissance est calculée dans la bande thêta pour les six canaux frontaux FPZ, F3, Fz, F4, AF3, AF4, et pour les trois canaux occipitaux O1, O2 et Oz et dans la bande alpha pour les quatre canaux pariétaux P3, Pz, P4, POz, et où

$$\#Theta\ frequency\ bins = (\mathrm{IAF} - 6 : \mathrm{IAF} - 2) \cdot \frac{1}{Epoch\ length}$$

et

$$\#Alpha\ frequency\ bins = (\mathrm{IAF} - 2 : \mathrm{IAF} + 2) \cdot \frac{1}{Epoch\ length}$$

**4.** Procédé selon l'une quelconque des revendications précédentes, **caractérisé en ce qu'**il comprend, après l'étape A et avant l'étape C, l'étape supplémentaire suivante :

G. filtrer (110) ledit signal EEG à travers au moins un filtre passe-bas, éventuellement dans la plage de fréquences de 0,1 Hz à 30 Hz, plus éventuellement de 1 Hz à 25 Hz, plus éventuellement encore de 2Hz à 20 Hz, plus éventuellement encore de 3 Hz à 15 Hz, plus éventuellement encore de 4 Hz à 12 Hz, et plus éventuellement encore à travers au moins un filtre de Butterworth de quatrième ordre.

**5.** Procédé selon l'une quelconque des revendications précédentes, **caractérisé en ce qu'**il comprend, après l'étape A et avant l'étape C, l'étape supplémentaire suivante :

H. éliminer un ou plusieurs artéfacts du signal EEG,

dans lequel ledit ou lesdits artéfacts sont choisis dans le groupe comprenant les artéfacts dus aux mouvements oculaires, les artéfacts dus aux mouvements du sujet dont l'état mental doit être estimé, les artéfacts dus à la saturation du signal EEG.

**6.** Procédé selon la revendication 5, **caractérisé en ce que** l'étape H élimine les artéfacts dus aux mouvements oculaires du signal EEG par le biais d'un signal d'électro-oculographie EOG qui est disponible à l'étape A (100), où le signal EOG est acquis ou a été acquis simultanément avec le signal EEG.

**7.** Procédé selon l'une quelconque des revendications précédentes, **caractérisé en ce que** l'étape C (130) calcule ladite densité spectrale de puissance à l'aide d'une fenêtre de Hanning, ayant éventuellement une durée égale à *Epoch length,* ou à l'aide d'une fenêtre en cosinus surélevé.

**8.** Procédé selon l'une quelconque des revendications précédentes, **caractérisé en ce que** ladite estimation dudit état mental est la fonction identité de la fonction discriminante linéaire *ytest(t),* en vertu de laquelle elle coïncide avec la fonction discriminante linéaire *ytest(t).*

**9.** Procédé selon l'une quelconque des revendications 1 à 7, **caractérisé en ce que** ladite estimation dudit état mental est un indice $W_{EEG}$ égal à une moyenne mobile de la fonction discriminante linéaire *ytest(t).*

**10.** Procédé selon la revendication 9, **caractérisé en ce que** ledit indice $W_{EEG}$ est égal à une moyenne mobile simple, éventuellement d'une durée égale à 4 fois la durée *Epoch length* des époques de la fonction discriminante linéaire *ytest (t) .*

**11.** Procédé selon la revendication 9, **caractérisé en ce que** ledit indice $W_{EEG}$ est égal à une moyenne mobile pondérée de la fonction discriminante linéaire *ytest(t).*

**12.** Appareil d'estimation d'un état mental d'un sujet lors de l'exécution d'une tâche, **caractérisé en ce qu'**il comprend une unité de traitement conçue pour exécuter le procédé d'estimation d'un état mental d'un sujet lors de l'exécution d'une tâche selon l'une quelconque des revendications 1 à 11.

**13.** Appareil selon la revendication 12, **caractérisé en ce qu'**il comprend en outre une ou plusieurs électrodes conçues pour être raccordées, éventuellement par l'intermédiaire de câbles, à l'unité de traitement et destinées à être placées sur un scalp pour acquérir des signaux EEG.

**14.** Programme informatique **caractérisé en ce qu'**il est adapté pour exécuter le procédé d'estimation d'un état mental d'un sujet lors de l'exécution d'une tâche selon l'une quelconque des revendications 1 à 11.

**15.** Support de mémoire lisible par ordinateur **caractérisé en ce qu'**il stocke le programme informatique selon la revendication 14.

EEG based workload Index ($W_{EEG}$)

8MA($y$)

170

160

Linear Discriminant Function ($y$)

$$y = \sum_i w \cdot f_i + b$$

130

Power Spectral Density (PSD)

140

Individual Alpha Frequency (IAF)

150

asSWLDA

120

Artifacts Correction

125

110

115

2 sec, shift 125 msec (EEG)

Channels

Time (sec)

100

EEG and EOG recording

*Fig. 1*

*Fig. 2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070173733 A

**Non-patent literature cited in the description**

- **E.A. BYRNE et al.** Psychophysiology and adaptive automation. *Biol. Psychol.,* 1996, vol. 42, 249-268
- **S. WELKE et al.** Single-Trial Detection of Cognitive Processes for Increasing Traffic Safety. *Proceedings of the 21st (Esv) International Technical Conference On The Enhanced Safety Of Vehicles,* June 2009
- Novel applications of BCI technology: Psychophysiological optimization of working conditions in industry. **B. VENTHUR et al.** IEEE International Conference on Systems Man and Cybernetics (SMC). IEEE, 2010, 417-421
- **B. WILLEMS.** *Air traffic control specialist visual scanning II: Task load, visual noise, and intrusions into controlled airspace,* 1999, http://www.academia.edu/3326249/Air_traffic_control_specialist_visual_scanning_II_Task_load_visual_noise_and_intrusions_into_controlled_airspace*
- **G.F. WILSON et al.** The use of cardiac and eye blink measures to determine flight segment in F4 crews. *Aviat. Space Environ. Med.,* 1991, vol. 62, 959-962
- **S.G. HART.** Development of NASA-TLX (Task Load Index): Results of Empirical and Theoretical Research. *Human Mental Workload,* 1988, 139-183
- **J.R. COMSTOCK.** *MATB - Multi-Attribute Task Battery for human operator workload and strategic behaviour research,* 1994
- **G. BORGHINI et al.** Measuring neurophysiological signals in aircraft pilots and car drivers for the assessment of mental workload, fatigue and drowsiness. *Neurosci. Biobehav. Rev.,* 2012
- **H.A. COLLE et al.** Double trade-off curves with different cognitive processing combinations: testing the cancellation axiom of mental workload measurement theory. *Hum. Factors,* 1999, vol. 41, 35-50

- Application of Research Techniques for Documenting Cognitive Processes in Air Traffic Control: Sector Complexity and Decision Making. **MOGFORD et al.** DOT/FAA/CT-TN94/3. Atlantic City International Airport: Federal Aviation Administration Technical Center, 1994
- **C. DUSSAULT et al.** EEG and ECG changes during selected flight sequences. *Aviat. Space Environ. Med.,* 2004, vol. 75, 889-897
- **T.C. HANKINS et al.** A comparison of heart rate, eye activity, EEG and subjective measures of pilot mental workload during flight. *Aviat. Space Environ. Med.,* 1998, vol. 69, 360-367
- **P. ARICÒ et al.** Towards a multimodal bioelectrical framework for the online mental workload evaluation. *36th Annual International Conference of the IEEE Engineering in Medicine and Biology Society (EMBC),* 2014, 3001-3004
- **J. KOHLMORGEN et al.** *Improving human performance in a real operating environment through real-time mental workload detection,* 2007
- **R.A. FISHER.** The Use of Multiple Measurements in Taxonomic Problems. *Annals of Eugenics,* 1936, vol. 7, 179-188
- **A. GEVINS et al.** Monitoring working memory load during computer-based tasks with EEG pattern recognition methods. *Hum. Factors,* 1998, vol. 40, 79-91
- **A.R. NIKOLAEV et al.** Reproducible EEG alpha-rhythm patterns in solving psychological tasks. *Fiziol. Cheloveka,* 1998, vol. 24, 5-12
- **N.R. DRAPER et al.** Applied Regression Analysis. Wiley-Interscience, 1998
- **J.C. CHRISTENSEN et al.** The effects of day-to-day variability of physiological data on operator functional state classification. *Neuroimage,* 2012, vol. 59, 57-63
- Cognitive Workload Gauge Development: Comparison of Real-time Classification Methods, Augmented Cognition: Past, Present and Future. **P.L. CRAVEN et al.** Foundation in Augmented Cognition. Strategic Analysis, Inc, 2006, 66-74

- **F. ALOISE et al.** A comparison of classification techniques for a gaze-independent P300-based brain-computer interface. *Journal of neural engineering,* 2012, vol. 9, 045012
- **D.J. KRUSIENSKI.** A comparison of classification techniques for the P300 Speller. *J. Neural Eng.,* 2006, vol. 3, 299-305
- **A. BURGESS et al.** Individual reliability of amplitude distribution in topographical mapping of EEG. *Electroencephalogr. Clin. Neurophysiol.,* 1993, vol. 86, 219-223
- **L.K. MCEVOY et al.** Test-retest reliability of cognitive EEG. *Clin. Neurophysiol.,* 2000, vol. 111, 457-463
- **V.E. POLLOCK et al.** Reliability of topographic quantitative EEG amplitude in healthy late-middle-aged and elderly subjects. *Electroencephalogr. Clin. Neurophysiol.,* 1991, vol. 79, 20-26
- **M.C. SALINSKY et al.** Test-retest reliability in EEG frequency analysis. *Electroencephalogr. Clin. Neurophysiol.,* 1991, vol. 79, 382-392
- **V.N. VAPNIK.** The Nature of Statistical Learning Theory. Springer, 2000
- **LUXBURG et al.** *Statistical Learning Theory: Models, Concepts, and Results,* 2008
- **V. JURCAK et al.** 10/20, 10/10, and 10/5 systems revisited: their validity as relative head-surface-based positioning systems. *NeuroImage,* 2007, vol. 34 (4), 1600-1611
- **GRATTON et al.** A new method for off-line removal of ocular artifact. *Electroencephalogr. Clin. Neurophysiol.,* 1983, vol. 55, 468-484
- **R. ELUL.** Gaussian behavior of the electroencephalogram: changes during performance of mental task. *Science,* 1969, vol. 164, 328-331
- **F.J. HARRIS.** On the use of windows for harmonic analysis with the discrete Fourier transform. *Proceedings of the IEEE,* 1978, vol. 66, 51
- **W. KLIMESCH.** EEG alpha and theta oscillations reflect cognitive and memory performance: a review and analysis. *Brain Res. Rev.,* 1999, vol. 29, 169-195
- **D. BAMBER.** The area above the ordinal dominance graph and the area below the receiver operating characteristic graph. *Journal of Mathematical Psychology,* 1975, vol. 12, 387-415